(19) Europäisches Patentamt
European Patent Office
Office européen des brevets

(11) EP 4 435 032 A1

(12) EUROPEAN PATENT APPLICATION

(43) Date of publication:
25.09.2024 Bulletin 2024/39

(21) Application number: 23163241.5

(22) Date of filing: 21.03.2023

(51) International Patent Classification (IPC):
*C08G 64/18* (2006.01) *C08G 64/42* (2006.01)

(52) Cooperative Patent Classification (CPC):
(C-Sets available)
**C08G 64/42; C08G 64/12; C08G 64/14; C08G 64/18; C08L 69/00** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **SHPP Global Technologies B.V.**
**4612 PX Bergen op Zoom (NL)**

(72) Inventors:
- **Chellamuthu, Manojkumar**
**Mount Vernon, 47620 (US)**
- **Mahood, James A.**
**Mount Vernon, 47620 (US)**
- **Hein, Christopher Luke**
**Mount Vernon, 47620 (US)**

(74) Representative: **Modiano, Gabriella Diana et al**
**Modiano & Partners (DE)**
**Steinsdorfstrasse, 14**
**80538 München (DE)**

(54) **BRANCHED POLYCARBONATE COPOLYMER COMPOSITIONS HAVING IMPROVED TOUGHNESS PROPERTIES AND METHODS FOR EVALUATING THE SAME**

(57) A thermoplastic composition includes: a first branched polycarbonate copolymer end-capped with 4-hydroxybenzonitrile (4-HBN) having a molecular weight M1; and a second branched polycarbonate copolymer end-capped with 4-HBN having a molecular weight M2. M1 is less than M2. Methods for determining entanglement molecular weight (Me) of a polycarbonate copolymer include: preparing a sample of a branched polycarbonate copolymer including a 1,1,1-tris-hydroxy-phenylethane (THPE) branching agent and that is end-capped with 4-HBN; determining phase angle (∂) versus complex modulus (G*) of the sample using a small amplitude oscillatory rheology process at a specified temperature; determining a minima in the ∂ of the sample; determining a plateau modulus ($G_N$) of the sample from the determined minima of ∂; and calculating the Me of the sample from the $G_N$.

Chemical Structure of CFR Polycarbonate Copolymer

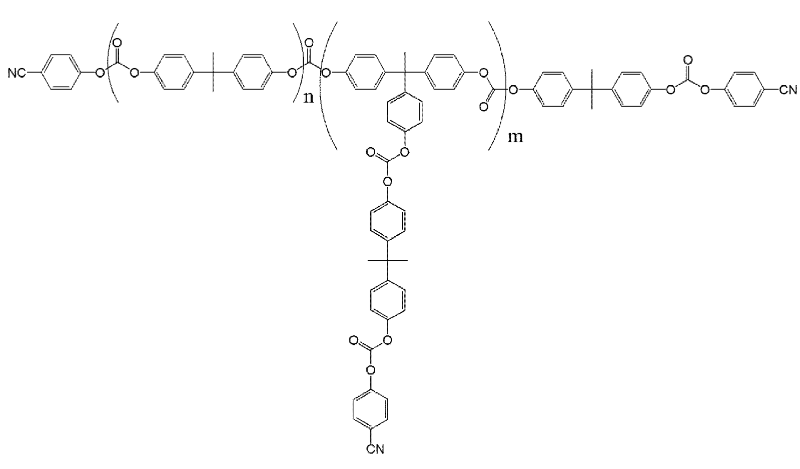

FIG. 1



Processed by Luminess, 75001 PARIS (FR)

(Cont. next page)

(52) Cooperative Patent Classification (CPC): (Cont.)

C-Sets
**C08L 69/00, C08L 69/00**

## Description

### FIELD OF THE DISCLOSURE

The present disclosure relates to branched polycarbonate (PC) copolymer compositions, and in particular to PC copolymer compositions having improved toughness properties.

### BACKGROUND OF THE DISCLOSURE

Transparent PC copolymer compositions having good flame properties and high ductility are desirable for use in numerous applications including consumer electronics, electrification, and display and lighting applications. While PC copolymer compositions having good transparency and flammability (anti-drip) properties have been developed, these compositions generally have lower ductility and/or heat distortion temperature (HDT) properties.

Commercial adoption of PC copolymer blends having a 4-hydroxybenzonitrile (4-HBN) end-cap and a 1,1,1-tris-hydroxy-phenylethane (THPE) branching agent has been limited to applications where V0 flame ratings are needed but impact strength is less important. Such compositions cannot satisfy basic toughness expectations for certain applications. Extruded profiles intended for lighting applications (e.g., tubes for fluorescent bulbs) including these conventional PC copolymer compositions are too brittle when cut to size. While acceptable extruded profiles could be achieved using a low cutting feed rate, such manufacturing restrictions are not feasible for commercial applications. It is anticipated that compositions possessing inherent toughness will allow for an increase in production yield and rate.

Other attempts to address the problem of impact retention have included use of PC-siloxane copolymers. These copolymers have good flame ratings and impact strength, but the siloxane content in the copolymer - which can be up to 20 wt% or more of the copolymer - results in opaque compositions. Further compositions blend PC-siloxane copolymer having a lower siloxane content (e.g., 6.5 wt% siloxane) with a small amount of a higher siloxane content PC-siloxane copolymer. Such compositions have improved toughness but undesirable color and optical effects such as high haze.

Gaming consoles and controllers are additional applications in which improved PC copolymer compositions could be useful. Gaming consoles have been shown to crack during normal use. Additionally, when resin was pigmented with light diffusers for decorative purposes, molded controller parts could not pass a standard toughness drop test.

These and other shortcomings are addressed by aspects of the present disclosure.

### SUMMARY

Aspects of the disclosure relate to thermoplastic compositions including: a first branched polycarbonate copolymer end-capped with 4-hydroxybenzonitrile (4-HBN) having a molecular weight M1; and a second branched polycarbonate copolymer end-capped with 4-HBN having a molecular weight M2. M1 is less than M2.

Aspects of the disclosure further relate to methods for determining entanglement molecular weight (Me) of a polycarbonate copolymer including: preparing a sample of a branched polycarbonate copolymer including a 1,1,1-tris-hydroxy-phenylethane (THPE) branching agent and that is end-capped with 4-HBN; determining phase angle ($\partial$) versus complex modulus (G*) of the sample using a small amplitude oscillatory rheology process at a specified temperature; determining a minima in the $\partial$ of the sample; determining a plateau modulus (GN) of the sample from the determined minima of $\partial$; and calculating the Me of the sample from the GN.

### BRIEF DESCRIPTION OF THE FIGURES

In the drawings, which are not necessarily drawn to scale, like numerals may describe similar components in different views. Like numerals having different letter suffixes may represent different instances of similar components. The drawings illustrate generally, by way of example, but not by way of limitation, various aspects discussed in the present document.

FIG. 1 shows the chemical structure of a CFR ("clear flame retardant") polycarbonate copolymer according to aspects of the present disclosure.

FIG. 2 is a graph of dynamic oscillatory rheology on a series of branched PC and PC copolymers with 4-hydroxybenzonitrile (4-HBN) end groups; the data was collected at a temperature of 170 °C.

FIG. 3 is a graph showing notched Izod impact (NII) strength values at 23 °C for PC copolymers and PC copolymer blends for comparative and example compositions according to aspects of the disclosure.

## DETAILED DESCRIPTION

Before the present compounds, compositions, articles, systems, devices, and/or methods are disclosed and described, it is to be understood that they are not limited to specific synthetic methods unless otherwise specified, or to particular reagents unless otherwise specified, as such can, of course, vary. It is also to be understood that the terminology used herein is for the purpose of describing particular aspects only and is not intended to be limiting.

Various combinations of elements of this disclosure are encompassed by this disclosure, e.g., combinations of elements from dependent claims that depend upon the same independent claim.

Moreover, it is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including: matters of logic with respect to arrangement of steps or operational flow; plain meaning derived from grammatical organization or punctuation; and the number or type of aspects described in the specification.

All publications mentioned herein are incorporated herein by reference to disclose and describe the methods and/or materials in connection with which the publications are cited.

### Definitions

It is also to be understood that the terminology used herein is for the purpose of describing particular aspects only and is not intended to be limiting. As used in the specification and in the claims, the term "comprising" can include the aspects "consisting of" and "consisting essentially of." Unless defined otherwise, all technical and scientific terms used herein have the same meaning as commonly understood by one of ordinary skill in the art to which this disclosure belongs. In this specification and in the claims which follow, reference will be made to a number of terms which shall be defined herein.

As used in the specification and the appended claims, the singular forms "a," "an" and "the" include plural referents unless the context clearly dictates otherwise. Thus, for example, reference to "a branched polycarbonate copolymer" includes mixtures of two or more branched polycarbonate copolymers.

As used herein, the term "combination" is inclusive of blends, mixtures, alloys, reaction products, and the like.

Ranges can be expressed herein as from one value (first value) to another value (second value). When such a range is expressed, the range includes in some aspects one or both of the first value and the second value. Similarly, when values are expressed as approximations, by use of the antecedent 'about,' it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "about" and "at or about" mean that the amount or value in question can be the designated value, approximately the designated value, or about the same as the designated value. It is generally understood, as used herein, that it is the nominal value indicated $\pm 10\%$ variation unless otherwise indicated or inferred. The term is intended to convey that similar values promote equivalent results or effects recited in the claims. That is, it is understood that amounts, sizes, formulations, parameters, and other quantities and characteristics are not and need not be exact, but can be approximate and/or larger or smaller, as desired, reflecting tolerances, conversion factors, rounding off, measurement error and the like, and other factors known to those of skill in the art. In general, an amount, size, formulation, parameter or other quantity or characteristic is "about" or "approximate" whether or not expressly stated to be such. It is understood that where "about" is used before a quantitative value, the parameter also includes the specific quantitative value itself, unless specifically stated otherwise.

Disclosed are the components to be used to prepare the compositions of the disclosure as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is

disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the disclosure. Thus, if there are a variety of additional steps that can be performed it is understood that each of these additional steps can be performed with any specific aspect or combination of aspects of the methods of the disclosure.

References in the specification and concluding claims to parts by weight of a particular element or component in a composition or article, denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

A weight percent of a component, unless specifically stated to the contrary, is based on the total weight of the formulation or composition in which the component is included.

As used herein, the terms "number average molecular weight" or "$M_n$" can be used interchangeably, and refer to the statistical average molecular weight of all the polymer chains in the sample and is defined by the formula:

$$M_n = \frac{\sum N_i M_i}{\sum N_i},$$

where $M_i$ is the molecular weight of a chain and $N_i$ is the number of chains of that molecular weight. $M_n$ can be determined for polymers, e.g., polycarbonate polymers, by methods well known to a person having ordinary skill in the art using molecular weight standards, e.g. polycarbonate standards or polystyrene standards, preferably certified or traceable molecular weight standards.

As used herein, the terms "weight average molecular weight" or "$M_w$" can be used interchangeably, and are defined by the formula:

$$M_w = \frac{\sum N_i {M_i}^2}{\sum N_i M_i},$$

where $M_i$ is the molecular weight of a chain and $N_i$ is the number of chains of that molecular weight. Compared to $M_n$, $M_w$ takes into account the molecular weight of a given chain in determining contributions to the molecular weight average. Thus, the greater the molecular weight of a given chain, the more the chain contributes to the $M_w$. $M_w$ can be determined for polymers, e.g., polycarbonate polymers, by methods well known to a person having ordinary skill in the art using molecular weight standards, e.g., polycarbonate standards or polystyrene standards, preferably certified or traceable molecular weight standards.

As used herein, the terms "polydispersity index" or "PDI" can be used interchangeably, and are defined by the formula:

$$PDI = \frac{M_w}{M_n}.$$

The PDI has a value equal to or greater than 1, but as the polymer chains approach uniform chain length, the PDI approaches unity.

As used herein, "polycarbonate" refers to an oligomer or polymer including residues of one or more dihydroxy compounds, e.g., dihydroxy aromatic compounds, joined by carbonate linkages; it also encompasses homopolycarbonates, copolycarbonates, and (co)polyester carbonates.

The terms "residues" and "structural units", used in reference to the constituents of the polymers, are synonymous throughout the specification.

As used herein the terms "weight percent," "wt%," and "wt. %," which can be used interchangeably, indicate the percent by weight of a given component based on the total weight of the composition, unless otherwise specified. That is, unless otherwise specified, all wt% values are based on the total weight of the composition. It should be understood that the sum of wt% values for all components in a disclosed composition or formulation are equal to 100.

Unless otherwise stated to the contrary herein, all test standards are the most recent standard in effect at the

time of filing this application.

Each of the raw materials used in example and/or comparative compositions described herein are either commercially available and/or the methods for the production thereof are known to those of skill in the art.

It is understood that the compositions disclosed herein have certain functions. Disclosed herein are certain structural requirements for performing the disclosed functions and it is understood that there are a variety of structures that can perform the same function that are related to the disclosed structures, and that these structures will typically achieve the same result.

The present disclosure relates to compositions including branched polycarbonate (PC) copolymers and to rheological methods to measure entanglement molecular weight/entanglement density of these copolymers. The methods described herein are particularly useful for polycarbonates with branched architectures exhibiting various concentrations of branching agent (1,1,1-tris-hydroxy-phenylethane (THPE)). A dynamic mechanical analysis method utilizing a small amplitude oscillatory rheology technique is used to measure plateau modulus. The plateau modulus is calculated from the minima in phase angle while plotting as a function of complex modulus. The minima in phase angle were obtained at a temperature range of 20-40 °C above the glass transition temperature of the polycarbonate copolymer.

Entanglement molecular weight (M*e*) and its inverse property entanglement density is calculated from the plateau modulus at a specific temperature within 20-40 °C above the glass transition temperature. The ductility (impact performance) of the composition may be predicted from the calculated entanglement density value.

Accordingly, aspects of the disclosure include methods for determining the magnitude of impact performance of the branched PC copolymers. The method includes: measuring phase angle minima at a temperature range of 20-40 °C above the glass transition temperature of the branched PC copolymer to calculate plateau modulus; determining the entanglement molecular weight from the plateau modulus and determining (predicting) impact/ductility performance based on the value. Entanglement density (molecular weight) vs impact performance is determined to be a function of the branched mol% content in the PC copolymers.

Molecular weight and branching architecture play an important role in determining the physical properties and processability of polymers. Plastics get their mechanical properties when the molecular weight of the polymers is raised above a certain critical molecular weight (M*c*). At molecular weights higher than M*c*, the polymer chains entangle and begin to exhibit enhanced mechanical properties. The onset of molecular entanglement is marked by an exponential increase in the low shear/zero shear viscosity. The entanglement molecular weight (M*e*) refers to the statistical average weight of the polymer chain between two entanglement points. Therefore, a highly entangled polymer will have a smaller M*e* as compared to a less entangled polymer.

The mechanical properties of a polymer do not increase indefinitely with it is molecular weight. The increase in mechanical performance plateaus after reaching a molecular weight for a given polymer. Since the viscosity of the polymer also increases with its molecular weight, an optimal molecular weight may be determined at which the desired mechanical properties can be achieved without compromising its processability.

Branching architecture in a polymer backbone affects the entanglement density for a given molecular weight. It also affects its physical properties. Branching includes long chain branching and short chain branching. Long chain branches are those whose molecular weight exceeds the Me and the branches themselves can entangle, impacting properties and processing.

## Thermoplastic Compositions

Aspects of the disclosure relate to thermoplastic compositions including: a first branched polycarbonate copolymer end-capped with 4-hydroxybenzonitrile (4-HBN) having a molecular weight M1; and a second branched polycarbonate copolymer end-capped with 4-HBN having a molecular weight M2. M1 is less than M2. Molecular weight is evaluated according to polycarbonate standards.

The branched polycarbonate copolymers are formed from polycarbonate according to conventional methods. The term polycarbonate as used herein is not intended to refer to only a specific polycarbonate or group of polycarbonates, but rather refers to the any one of the class of compounds containing a repeating chain of carbonate groups. In one aspect, a polycarbonate can include any one or more of those polycarbonates disclosed and described in U.S. Patent No. 7,786,246, which is hereby incorporated by reference in its entirety for the specific purpose of disclosing various polycarbonate compositions and methods for manufacture of same.

In one aspect, a polycarbonate polymer as disclosed herein can be an aliphaticdiol based polycarbonate. In another aspect, the polycarbonate polymer can include a carbonate unit derived from a dihydroxy compound, such as, for example, a bisphenol that differs from the aliphatic diol. In still further aspects, an exemplary polycarbonate polymer includes aromatic polycarbonates conventionally manufactured through a transesterification reaction of one or more aromatic dihydroxy compound(s) and a carbonic acid diester in the presence of one or more catalyst(s).

In one aspect, non-limiting examples of suitable bisphenol compounds include the following: 4,4'-dihydroxybiphenyl, 1,6-dihydroxynaphthalene, 2,6-dihydroxynaphthalene, bis(4-hydroxyphenyl)methane, bis(4-hydroxyphenyl)diphenylmethane, bis(4-hydroxyphenyl)-1-naphthylmethane, 1,2-bis(4-hydroxyphenyl)ethane, 1,1-bis(4-hydroxyphenyl)-1-phenylethane, 2-(4-hydroxyphenyl)-2-(3-hydroxyphenyl)propane, bis(4-hydroxyphenyl)phenylmethane, 2,2-bis(4-hydroxy-3-bromophenyl)propane, 1,1-bis(hydroxyphenyl)cyclopentane, 1,1-bis(4-hydroxyphenyl)cyclohexane, 1,1-bis(4-hydroxy-3 methylphenyl)cyclohexane 1,1-bis(4-hydroxyphenyl)isobutene, 1,1-bis(4-hydroxyphenyl)cyclododecane, trans-2,3-bis(4-hydroxyphenyl)-2-butene, 2,2-bis(4-hydroxyphenyl)adamantine, (alpha, alpha'-bis(4-hydroxyphenyl)toluene, bis(4-hydroxyphenyl)acetonitrile, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl)propane, 2,2-bis(3-sec-butyl-4-hydroxyphenyl)propane, 2,2-bis(3-t-butyl-4-hydroxyphenyl)propane, 2,2-bis(3-cyclohexyl-4-hydroxyphenyl)propane, 2,2-bis(3-allyl-4-hydroxyphenyl)propane, 2,2-bis(3-methoxy-4-hydroxyphenyl)propane, 2,2-bis(4-hydroxyphenyl)hexafluoropropane, 1,1-dichloro-2,2-bis(4-hydroxyphenyl)ethylene, 1,1-dibromo-2,2-bis(4-hydroxyphenyl)ethylene, 1,1-dichloro-2,2-bis(5-phenoxy-4-hydroxyphenyl)ethylene, 4,4'-dihydroxybenzophenone, 3,3-bis(4-hydroxyphenyl)-2-butanone, 1,6-bis(4-hydroxyphenyl)-1,6-hexanedione, ethylene glycol bis(4-hydroxyphenyl)ether, bis(4-hydroxyphenyl)ether, bis(4-hydroxyphenyl) sulfide, bis(4-hydroxyphenyl)sulfoxide, bis(4-hydroxyphenyl)sulfone, 9,9-bis(4-hydroxyphenyl)fluorene, 2,7-dihydroxypyrene, 6,6'-dihydroxy-3,3,3',3'-tetramethylspiro(bis)indane ("spirobiindane bisphenol"), 3,3-bis(4-hydroxyphenyl)phthalide, 2,6-dihydroxydibenzo-p-dioxin, 2,6-dihydroxythianthrene, 2,7-dihydroxyphenoxathin, 2,7-dihydroxy-9,10-dimethylphenazine, 3,6-dihydroxydibenzofuran, 3,6-dihydroxydibenzothiophene, and 2,7-dihydroxycarbazole, and the like, as well as combinations including at least one of the foregoing dihydroxy aromatic compounds.

In another aspect, exemplary bisphenol compounds can include 1,1-bis(4-hydroxyphenyl)methane, 1,1-bis(4-hydroxyphenyl)ethane, 2,2-bis(4-hydroxyphenyl)propane (hereinafter "bisphenol A" or "BPA"), 2,2-bis(4-hydroxyphenyl)butane, 2,2-bis(4-hydroxyphenyl)octane, 1,1-bis(4-hydroxyphenyl)propane, 1,1-bis(4-hydroxyphenyl)n-butane, 2,2-bis(4-hydroxy-1-methylphenyl)propane, 1,1-bis(4-hydroxy-t-butylphenyl)propane, 3,3-bis(4-hydroxyphenyl)phthalimidine, 2-phenyl-3,3-bis(4-hydroxyphenyl)phthalimidine ("PPPBP"), and 9,9-bis(4-hydroxyphenyl)fluorene. BPA (CAS No. 80-07-7) has the following chemical structure:

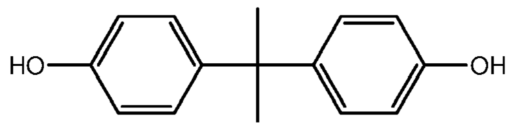

Combinations including at least one dihydroxy aromatic compound can also be used. In another aspect, other types of diols can be present in the polycarbonate.

Branched polycarbonate blocks can be prepared by adding a branching agent during polymerization. These branching agents include polyfunctional organic compounds containing at least three functional groups selected from hydroxyl, carboxyl, carboxylic anhydride, haloformyl, and mixtures of the foregoing functional groups. Specific examples include trimellitic acid, trimellitic anhydride, trimellitic trichloride, tris-p-hydroxy phenyl ethane, isatin-bis-phenol, tris-phenol TC (1,3,5-tris((p-hydroxyphenyl)isopropyl)benzene), tris-phenol PA (4(4(1,1-bis(p-hydroxyphenyl)-ethyl) alpha, alpha-dimethylbenzyl)phenol), 4-chloroformyl phthalic anhydride, trimesic acid, and benzophenone tetracarboxylic acid. In a specific aspect the branching agent is 1,1,1-tris-hydroxy-phenylethane (THPE). THPE (CAS No. 27955-94-8) has the following chemical structure:

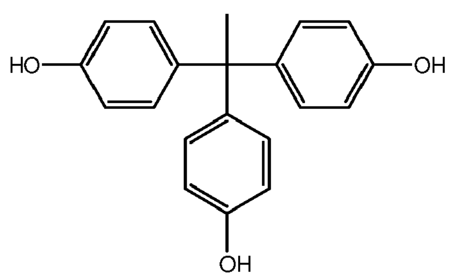

The branching agents can be added at a level of about 0.25 wt% to about 5.0 wt%, or in specific aspects at a level of about 0.25 wt% to about 4.0 wt%, or 0.25 wt% to about 3.0 wt%, or 0.4 wt% to about 5.0 wt%, or 0.4 wt% to about 4.0 wt%, or 0.4 wt% to about 3.0 wt%, or 0.5 wt% to about 5 wt%, or 0.5 wt% to about 4.0 wt%, or 0.5 wt% to about 3.0 wt%.

Polycarbonates can be manufactured by processes such as interfacial polymerization and melt polymerization. Although the reaction conditions for interfacial polymerization can vary, an exemplary process generally involves dissolving or dispersing a dihydric phenol reactant in aqueous caustic soda or potash, adding the resulting mixture to a suitable water-immiscible solvent medium, and contacting the reactants with a carbonate precursor in the presence of a catalyst such as triethylamine or a phase transfer catalyst, under controlled pH conditions, e.g., about 8 to about 10. The most commonly used water immiscible solvents include methylene chloride, 1,2-dichloroethane, chlorobenzene, toluene, and the like.

All types of polycarbonate end groups are contemplated as being useful in the polycarbonates described herein, provided that such end groups do not significantly adversely affect desired properties of the compositions.

An end-cap agent (also referred to as a chain stopper or a capping agent) can be included during polymerization. The end-cap agent limits molecular weight growth rate, and so controls molecular weight in the polycarbonate. Exemplary end-cap agents include certain mono-phenolic compounds, mono-carboxylic acid chlorides, and/or mono-chloroformates. Mono-phenolic end-cap agents are exemplified by monocyclic phenols such as phenol and $C_{1}$-$C_{22}$ alkyl-substituted phenols such as p-cumyl-phenol, resorcinol monobenzoate, and p- and tertiary-butyl phenol; and monoethers of diphenols, such as p-methoxyphenol. Alkyl-substituted phenols with branched chain alkyl substituents having 8 to 9 carbon atoms can be specifically mentioned. Certain mono-phenolic UV absorbers can also be used as a capping agent, for example 4-substituted-2-hydroxybenzophenones and their derivatives, aryl salicylates, monoesters of diphenols such as resorcinol monobenzoate, 2-(2-hydroxyaryl)-benzotriazoles and their derivatives, 2-(2-hydroxyaryl)-1,3,5-triazines and their derivatives, and the like.

Mono-carboxylic acid chlorides can also be used as end-cap agents. These include monocyclic, mono-carboxylic acid chlorides such as benzoyl chloride, $C_{1-22}$ alkyl-substituted benzoyl chloride, toluoyl chloride, halogen-substituted benzoyl chloride, bromobenzoyl chloride, cinnamoyl chloride, 4-nadimidobenzoyl chloride, and combinations thereof; polycyclic, mono-carboxylic acid chlorides such as trimellitic anhydride chloride, and naphthoyl chloride; and combinations of monocyclic and polycyclic mono-carboxylic acid chlorides. Chlorides of aliphatic monocarboxylic acids with less than or equal to about 22 carbon atoms are useful. Functionalized chlorides of aliphatic monocarboxylic acids, such as acryloyl chloride and methacryoyl chloride, are also useful. Also useful are mono-chloroformates including monocyclic, mono-chloroformates, such as phenyl chloroformate, alkyl-substituted phenyl chloroformate, p-cumyl phenyl chloroformate, toluene chloroformate, and combinations thereof.

In specific aspects the branched polycarbonate copolymer is end-capped with 4-hydroxybenzonitrile (4-HBN). 4-HBN (CAS No. 767-00-0) has the following chemical structure:

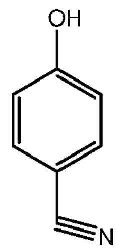

The end-capping agent, e.g., 4-HBN, may be present in the polycarbonate copolymer in an amount from 1-8 mol%. In particular aspects the end-capping agent is present in the polycarbonate copolymer in an amount of at least 1 mol%, or at least 2 mol%, or less than 8 mol%, or less than 7 mol%, or less than 6 mol%, or less than 5 mol%, or less than 4 mol%, or less than 3 mol%, or about 2 mol%.

FIG. 1 shows the chemical structure of a branched polycarbonate copolymer end-capped with 4-hydroxybenzonitrile (4-HBN) and including a 1,1,1-tris-hydroxy-phenylethane (THPE) branching agent according to aspects of the disclosure.

In some aspects the branched polycarbonate copolymer has a molecular weight of from about 20,000 grams per mole (g/mol) to about 60,000 g/mol. In specific aspects thermoplastic compositions according to aspects of the disclosure include a first branched polycarbonate copolymer having a Mw of from 20,000 grams per mole (g/mol) to less than 35,000 g/mol, and a second branched polycarbonate copolymer having a Mw of from 35,000 g/mol to 60,000 g/mol.

In certain aspects thermoplastic compositions according to aspects of the disclosure include at least 40 wt% of the first branched polycarbonate copolymer and the second branched polycarbonate copolymer, In further aspects the compositions include at least 45 wt%, at least 50 wt%, from 40-70 wt%, from 40-65 wt%, from 40-60 wt%, from 40-55 wt%, from 40-50 wt%, from 45-70 wt%, from 45-65 wt%, from 45-60 wt%, from 45-55 wt%, from 45-50 wt%, from 50-70 wt%, from 50-65 wt%, from 50-60 wt%, or from 50-55 wt%, of the first branched polycarbonate copolymer and the second branched polycarbonate copolymer.

In some aspects the thermoplastic composition further includes at least one additional polycarbonate component including a polycarbonate homopolymer, a blow molding polycarbonate, an additional polycarbonate copolymer, or a combination thereof.

In specific aspects the thermoplastic composition further includes (1) a third branched polycarbonate copolymer end-capped with 4-HBN, and (2) a polycarbonate homopolymer or an additional polycarbonate copolymer. In certain aspects the thermoplastic composition includes at least 90 wt% of the first branched polycarbonate copolymer, the second branched polycarbonate copolymer, the third branched copolymer end-capped with 4-HBN, and the polycarbonate homopolymer or additional polycarbonate copolymer. In further aspects the thermoplastic composition includes at least 91 wt%, or at least 92 wt%, or at least 93 wt%, or at least 94 wt%, or at least 95 wt%, or at least 96 wt%, or at least 97 wt%, or at least 98 wt%, or at least 99 wt%, of the first branched polycarbonate copolymer, the second branched polycarbonate copolymer, the third branched copolymer end-capped with 4-HBN, and the polycarbonate homopolymer or additional polycarbonate copolymer.

In some aspects one or more of the first branched polycarbonate copolymer, the second branched polycarbonate copolymer, and the at least one additional polycarbonate component include a 1,1,1-tris-hydroxy-phenylethane (THPE) branching agent. Each of these components may individually include the THPE branching agent at a level of about 0.25 wt% to about 5.0 wt%, or in specific aspects at a level of about 0.25 wt% to about 4.0 wt%, or 0.25 wt% to about 3.0 wt%, or 0.4 wt% to about 5.0 wt%, or 0.4 wt% to about 4.0 wt%, or 0.4 wt% to about 3.0 wt%, or 0.5 wt% to about 5 wt%, or 0.5 wt% to about 4.0 wt%, or 0.5 wt% to about 3.0 wt%.

Thermoplastic compositions according to aspects of the disclosure may further include at least one additional additive, including but not limited to, a flame retardant (FR) additive, a release agent, a heat stabilizer, a light stabilizer, a light diffuser, an anti-drip agent, a filler, or a combination thereof. Exemplary light diffusers may include PMMA, cross-linked siloxane (Tospearl), titanium dioxide, zinc sulfide, PTFE, barium sulfate, and combinations thereof. An exemplary filler includes aluminum metallic flakes.

Each of the first branched polycarbonate copolymer or the second branched polycarbonate copolymer may in some aspects have an entanglement density in a range of from $2\times10^{-4}$ moles per cubic centimeter ($mol/cm^3$) to $10\times10^{-4}$ $mol/cm^3$, or in further aspects from $3\times10^{-4}$ $mol/cm^3$ to $10\times10^{-4}$ $mol/cm^3$, or from $4\times10^{-4}$ $mol/cm^3$ to $10\times10^{-4}$ $mol/cm^3$, or from $2\times10^{-4}$ $mol/cm^3$ to $9\times10^{-4}$ $mol/cm^3$, or from $2\times10^{-4}$ $mol/cm^3$ to $8\times10^{-4}$ $mol/cm^3$, or from $2\times10^{-4}$ $mol/cm^3$ to $7\times10^{-4}$ $mol/cm^3$, or from $2\times10^{-4}$ $mol/cm^3$ to $6.5\times10^{-4}$ $mol/cm^3$, or from $3\times10^{-4}$ $mol/cm^3$ to $6.5\times10^{-4}$ $mol/cm^3$, or from $4\times10^{-4}$ $mol/cm^3$ to $6.5\times10^{-4}$ $mol/cm^3$.

Each of the first branched polycarbonate copolymer or the second branched polycarbonate copolymer may in some aspects have a plateau modulus of from 1.0 to 4.0 megapascals (MPa), or in further aspects from 1.0 to 3.0 MPa, or from 1.0 to 2.5 MPa, or from 1.5 to 4.0 MPa, or from 1.5 to 3.0 MPa, or from 1.5 to 2.5 MPa, or from 1.7 to 4.0 MPa, or from 1.7 to 3.0 MPa, or from 1.7 to 2.5 MPa.

Each of the first branched polycarbonate copolymer or the second branched polycarbonate copolymer may in some aspects have an entanglement molecular weight of from 1500 to 5000 g/mol, or in further aspects from 1500 to 4500 g/mol, or from 1500 to 4000 g/mol, or from 1500 to 3500 g/mol, or from 1500 to 3000 g/mol, or from 1500 to 2500 g/mol.

In some aspects a molded sample of the composition has a total light transmittance of at least 90% and a haze of 2% or less as tested in accordance with ASTM D1003 using a sample having a thickness of 3.2 mm.

### Methods for Determining M*e* of a Polycarbonate Copolymer

Aspects of the disclosure further relate to methods for determining entanglement molecular weight (M*e*) of a polycarbonate copolymer, including: preparing a sample of a branched polycarbonate copolymer including a 1,1,1-tris-hydroxy-phenylethane (THPE) branching agent and that is end-capped with 4-hydroxybenzonitrile (4-HBN); determining phase angle ($\partial$) versus complex modulus (G*) of the sample using a small amplitude oscillatory rheology process at a specified temperature; determining a minima in the $\partial$ of the sample; determining a plateau modulus ($G_N$) of the sample from the determined minima of $\partial$; and calculating the M*e* of the sample from the $G_N$.

The small amplitude oscillatory rheology process includes in some aspects: melting the sample at 300 °C in a rheometer (such as an ARES G2 rheometer); compressing the melted sample between parallel plates having a 1 millimeter (mm) gap; cooling the parallel plates in the rheometer to the specified temperature, wherein the specified temperature is at least 15 °C above a glass transition temperature (Tg) of the branched polycarbonate copolymer; and determining the phase angle ($\partial$) versus complex modulus (G*) of the sample at the specified temperature. A temperature range of 20-40 °C above the Tg may be selected to achieve minima in the phase angle while measuring frequency sweeps from 0.1 to 500 rad/s. In a specific aspect the specified temperature is 170 °C. This temperature may be selected to avoid compliance issues due to the stiffness acquired by the sample when measured close to its glass transition temperature.

Aspects of the method further include predicting impact strength of the sample by comparing the M*e* of the sample to a reference table showing M*e* versus notched Izod impact strength for a plurality of branched polycarbonate copolymers including a 1,1,1-tris-hydroxy-phenylethane (THPE) branching agent and that are end-capped with 4-hydroxybenzonitrile (4-HBN). The reference table may be generated by preparing a series of polycarbonate copolymers containing various amounts of THPE branching agent and 4-HBN end-cap content, determining the NII strength of each

copolymer, and including these NII strength values in a table to create the reference table.

In some aspects of the method the prepared branched polycarbonate copolymer has a molecular weight of from 40,000 to 50,000 g/mol. In further aspects the branched polycarbonate copolymer includes from 0.5 to 1 mole percent THPE and has a 4-HBN end-cap level 2 mol% or less.

**Articles of Manufacture**

In certain aspects, the present disclosure pertains to shaped, formed, or molded articles including the thermoplastic compositions described herein. The thermoplastic compositions can be molded into useful shaped articles by a variety of means such as injection molding, extrusion, rotational molding, blow molding and thermoforming to form articles and structural components of, for example, personal or commercial electronics devices, including but not limited to cellular telephones, tablet computers, personal computers, notebook and portable computers, and other such equipment, medical applications, RFID applications, automotive applications, and the like. In a further aspect, the article is extrusion molded. In a still further aspect, the article is injection molded.

In particular aspects the article is a component of a consumer electronics or lighting appliance device. In specific aspects the article is a signage component or a component of a backlit button for a consumer electronics device.

Various combinations of elements of this disclosure are encompassed by this disclosure, e.g., combinations of elements from dependent claims that depend upon the same independent claim.

**Aspects of the Disclosure**

In various aspects, the present disclosure pertains to and includes at least the following aspects.

Aspect 1. A thermoplastic composition comprising:

a first branched polycarbonate copolymer end-capped with 4-hydroxybenzonitrile (4-HBN) having a molecular weight M1; and
a second branched polycarbonate copolymer end-capped with 4-HBN having a molecular weight M2,
wherein M1 is less than M2, and wherein molecular weight is evaluated according to polycarbonate standards.

Aspect 2. The thermoplastic composition according to Aspect 1, wherein M1 is in a range of from 20,000 grams per mole (g/mol) to less than 35,000 g/mol, and wherein M2 is in a range of from 35,000 g/mol to 60,000 g/mol.

Aspect 3. The thermoplastic composition according to Aspect 1 or 2, wherein the composition comprises at least 40 wt% of the first branched polycarbonate copolymer and the second branched polycarbonate copolymer, wherein the combined weight percent value of all components does not exceed 100 wt%, and all weight percent values are based on the total weight of the composition.

Aspect 4. The thermoplastic composition according to any of Aspects 1-3, wherein each of the first branched polycarbonate copolymer and the second branched polycarbonate copolymer independently comprise from 1-8 mol% 4-HBN content.

Aspect 5. The thermoplastic composition according to any of Aspects 1 to 4, wherein the composition further comprises at least one additional polycarbonate component comprising a polycarbonate homopolymer, a blow molding polycarbonate, an additional polycarbonate copolymer, or a combination thereof.

Aspect 6. The thermoplastic composition according to any of Aspects 1 to 5, wherein the composition further comprises (1) a third branched polycarbonate copolymer end-capped with 4-HBN, and (2) a polycarbonate homopolymer or an additional polycarbonate copolymer.

Aspect 7. The thermoplastic composition according to Aspect 6, wherein the composition comprises at least 90 wt% of the first branched polycarbonate copolymer, the second branched polycarbonate copolymer, the third branched copolymer end-capped with 4-HBN, and the polycarbonate homopolymer or additional polycarbonate copolymer.

Aspect 8. The thermoplastic composition according to any of Aspects 1 to 7, wherein one or more of the first branched polycarbonate copolymer, the second branched polycarbonate copolymer, and the at least one additional polycarbonate component comprise a 1,1,1-tris-hydroxy-phenylethane (THPE) branching agent.

Aspect 9. The thermoplastic composition according to any of Aspects 1 to 8, wherein the one or more of the first branched polycarbonate copolymer, the second branched polycarbonate copolymer, and the at least one additional polycarbonate component comprise from 0.25 wt% to 5 mol% of the THPE branching agent.

Aspect 10. The thermoplastic composition according to any of Aspects 1 to 9, wherein the composition comprises at least one additional additive comprising a flame retardant (FR) additive, a release agent, a heat stabilizer, a light stabilizer, a light diffuser, an anti-drip agent, a filler, or a combination thereof.

Aspect 11. The thermoplastic composition according to any of Aspects 1 to 10, wherein the first branched polycarbonate copolymer or the second branched polycarbonate copolymer comprises:

an entanglement density in a range of from 2×$10^{-4}$ moles per cubic centimeter (mol/$cm^3$) to 10×$10^{-4}$ mol/$cm^3$;
a plateau modulus of from 1.0 to 4.0 megapascals (MPa); or
an entanglement molecular weight of from 1500 to 5000.

Aspect 12. The thermoplastic composition according to any of Aspects 1 to 11, wherein a molded sample of the composition has a total light transmittance of at least 90% and a haze of 2% or less as tested in accordance with ASTM D1003 using a sample having a thickness of 3.2 mm.

Aspect A method for determining entanglement molecular weight (Me) of a polycarbonate copolymer, comprising:

preparing a sample of a branched polycarbonate copolymer comprising a 1,1,1-tris-hydroxy-phenylethane (THPE) branching agent and that is end-capped with 4-hydroxybenzonitrile (4-HBN);
determining phase angle (∂) versus complex modulus (G*) of the sample using a small amplitude oscillatory rheology process at a specified temperature;
determining a minima in the ∂ of the sample;
determining a plateau modulus ($G_N$) of the sample from the determined minima of ∂; and
calculating the Me of the sample from the $G_N$.

Aspect 14. The method according to Aspect 13, wherein the small amplitude oscillatory rheology process comprises:

melting the sample at 300 °C in a rheometer;
compressing the melted sample between parallel plates having a 1 millimeter (mm) gap;
cooling the parallel plates in the rheometer to the specified temperature, wherein the specified temperature is at least 15 °C above a glass transition temperature (Tg) of the branched polycarbonate copolymer; and
determining the phase angle (∂) versus complex modulus (G*) of the sample at the specified temperature.

Aspect 15. The method according to Aspect 13 or 14, wherein the specified temperature is 170 °C.

Aspect 16. The method according to any of Aspects 13 to 15, further comprising predicting impact strength of the sample by comparing the Me of the sample to a reference table showing Me versus notched Izod impact strength for a plurality of branched polycarbonate copolymers comprising a 1,1,1-tris-hydroxy-phenylethane (THPE) branching agent and that are end-capped with 4-hydroxybenzonitrile (4-HBN).

Aspect 17. The method according to any of Aspects 13 to 16, wherein the specified temperature is from 20-40 °C above the Tg of the branched polycarbonate copolymer.

Aspect 18. The method according to any of Aspects 13 to 17, wherein the polycarbonate copolymer has a molecular weight of from 40,000 to 50,000 g/mol.

Aspect 19. The method according to any of Aspects 13 to 18, wherein the branched polycarbonate copolymer comprises from 0.5 to 1 mole percent THPE and has a 4-HBN end-cap level 2 mol% or less.

Aspect 20. An article comprising the thermoplastic composition according to any of Aspects 1 to 12.

Aspect 21. The article according to Aspect 20, wherein the article is a component of a consumer electronics or lighting appliance device.

Aspect 22. The article according to Aspect 21, wherein the article is a signage component or a component of a backlit button for a consumer electronics device.

## EXAMPLES

The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric. Unless indicated otherwise, percentages referring to composition are in terms of wt%.

There are numerous variations and combinations of reaction conditions, e.g., component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

Polycarbonate components according to Table 1 were tested and/or included in the example and comparative

compositions described herein:

**Table 1 - Polycarbonate Components**

| Component | Description | Molecular weight (Mw) | THPE Branching agent content (mol%) | Reactive 4-HBN end-cap content (mol%) |
|---|---|---|---|---|
| PC-1 | 100 grade linear PC homopolymer | 28,000 | 0 | 0 |
| PC-2 | High flow linear PC homopolymer | 22,000 | 0 | 0 |
| BEPC | Blow molding branched PC homopolymer with PC end-cap | 36,000 | 0.42 | 0 |
| CFR-PC | Branched PC copolymer | 28,000 | 3 | 6 |
| CFR-PC1 | Branched High Mw PC copolymer | 55,000 | 0.5 | 2 |
| CFR-PC2 | Branched High Mw PC copolymer | 40,000 | 1 | 2 |
| CFR-PC3 | Branched PC copolymer | 20,000 | 1 | 6 |

Each of these polycarbonate components were sourced from SABIC. CFR refers to a "clear flame retardant" PC copolymer.

Small Amplitude oscillatory shear (SAOS) rheology measurements were conducted using parallel plate geometry (25mm), gap approximately 1 m. To determine the linear viscoelastic region, strain sweep measurements were performed before the frequency sweep tests at SAOS. To calculate entanglement molecular weight (M*e*) frequency sweeps were investigated over a temperature range of 20-40 °C above glass transition temperature (Tg) to achieve the minima in the phase angle. The plateau modulus, $G_N$ was calculated from the minima in the phase angle. For the tested resins, samples were initially loaded at 300 °C for a time of 360 seconds before lowering the temperature to the evaluation temperature to measure the minima in the phase angle. It is noted that if the resin were tested at temperatures well above Tg (e.g., Tg + 100 °C), the minima in the phase angle would never be achieved.

ASTM Standard Multiaxial and IZOD Impact tests were used to analyze the mechanical properties. Multiaxial impact tests were conducted for ten samples per material according to ASTM D3763 under a crosshead speed of 50 millimeters per minute (mm/min) at room temperature (23 °C). Notched Izod impact strength was evaluated according to ASTM D256. Ten samples of each polymer were tested to get the average value of the impact strength. The samples were notched and tested under a 5.5 Joule (J) pendulum energy at a temperature of 23 °C. Transmission and haze were determined in accordance with ASTM D1003 using a sample having a thickness of 3.2 millimeters (mm).

A graph of dynamic rheology measurements on a series of branched copolymer resins with 2-6 mol% of 4-HBN end groups is shown in FIG. 2, and includes a plot of the phase angle (in degrees) against the complex modulus (in kPa). The minima of the phase angle for each polymer corresponds to plateau modulus, whose value is dependent on the molecular entanglement present in the polymer. Using the obtained plateau moduli and the equation below, the entanglement molecular weight was calculated for the resins.

$$M_e = \frac{\rho.R.T}{G_N^0}$$

Where, $G_N^0$ is the plateau modulus in MPa, $\rho$ is the polymer density (in grams per cubic centimeter, g/cm$^3$), *R* is the ideal gas constant (8.314 Joules per Kelvin·mol, J/K·mol) and T is the temperature (in degrees Kelvin, °K). A value of 1 gram per cubic centimeter (1 g/cm$^3$) was used for polymer density ($\rho$). Mechanical properties (NII and ductility), the calculated M*e*, and entanglement density values for each of the polymers is shown in Table 2:

**Table 2 - Properties of Table 1 PC Polymers**

| | PC-1 | PC-2 | CFR-PC | BEPC | CFR-PC 1 | CFR-PC 2 | CFR-PC 3 |
|---|---|---|---|---|---|---|---|
| Mw (g/mol) | 28,000 | 22,000 | 28,000 | 36,000 | 55,000 | 40,000 | 20,000 |
| THPE branching agent content (mol%) | 0 | 0 | 3 | 0.42 | 0.5 | 1 | 1 |
| Reactive 4-HBN end-cap content (mol%) | 0 | 0 | 6 | 0 | 2 | 2 | 6 |
| Plateau Modulus (MPa) | 1.9 | 2 | 1 | 1.8 | 2.3 | 1.9 | 1 |
| Entanglement Mw (M*e*) (g/mol) | 1910 | 1850 | 4000 | 2200 | 1600 | 1800 | 4100 |
| Entanglement Density (x$10^{-4}$ mol/$cm^3$) | 5.2 | 5.4 | 2.5 | 4.5 | 6.2 | 5.5 | 2.4 |
| Notched Izod impact (NII) strength (J/m at 23 °C) | 800 | 740 | 110 | 795 | 755 | 740 | 70 |
| Ductility (%) | 100 | 100 | 0 | 100 | 100 | 100 | 0 |

Entanglement density is calculated as the inverse of entanglement molecular weight. The notched Izod impact results shown in Table 2 above were surprising in that good toughness could be achieved as long as the polycarbonate copolymer has a MW of at least 40,000 g/mol with a THPE branching content 0.5 to 1 mol%.

Notched Izod impact values for several of the resins and blends of these resins are shown graphically in FIG. 3. It is observed that PC copolymers/PC copolymer blends including PC components with a lower entanglement molecular weight (PC-1, BEPC) have a much higher NII strength as compared to those including a PC component with a higher entanglement molecular weight (CFR-PC).

Comparative and example compositions including a combination of the PC polymers were prepared and tested as shown in Table 3:

**Table 3 - Example and Comparative Compositions Including a Combination of PC Polymers**

| **Components:** | **CE1** | **E1** | **E2** | **E3** |
|---|---|---|---|---|
| CFR-PC1 | | 30 | | 30 |
| CFR-PC2 | | | 30 | |
| CFR-PC3 | | 20 | 20 | |
| CFR-PC | 50 | | | 20 |
| BEPC | 28 | 28 | 28 | 28 |
| PC-1 | 19.15 | 19.15 | 19.15 | 19.15 |
| PC-2 | 2 | 2 | 2 | 2 |
| Rimar salt (FR additive) | 0.08 | 0.08 | 0.08 | 0.08 |
| Polymethylphenyl siloxane (FR synergist) | 0.4 | 0.4 | 0.4 | 0.4 |
| Pentaerythritol tetrastearate (PETS) | 0.27 | 0.27 | 0.27 | 0.27 |
| Cyclotetrasiloxane (FR synergist) | 0.1 | 0.1 | 0.1 | 0.1 |
| **Total:** | 100 | 100 | 100 | 100 |
| | | | | |
| **Properties:** | | | | |
| Heat distortion temperature (HDT) (°C) | 137 | 140 | 139 | 139 |
| NII Ductility (%) | 0 | 100 | 100 | 100 |
| Melt volume rate (MVR) ($cm^3$/10min) | 4.41 | 2.71 | 3.57 | 1.23 |

(continued)

| Properties: | | | | |
|---|---|---|---|---|
| Light transmittance (%) | 91 | 91 | 91 | 91 |
| Haze (%) | 2 | 2 | 1.2 | 1.8 |
| Plateau Modulus (MPa) | 1 | 1.4 | 1.8 | 1.6 |
| Entanglement MW (Me) (g/mol) | 3680 | 2630 | 2040 | 2300 |
| Entanglement density ($\times 10^{-4}$ mol/$cm^3$) | 2.7 | 3.8 | 5 | 4.4 |
| P(FTP) V0 (2 mm) | Pass | | | Pass |

Comparative composition CE1 included CFR-PC (Mw 28,000) with a THPE branch content of 3 mol% and had poor (0%) ductility, even with a high 4-HBN end-cap content (6 mol%). Compositions corresponding to CE1 have been used in applications requiring V0 (1 mm) flame performance to make thin wall parts. However, these compositions have low toughness (below PC standards).

Example compositions E1-E3 each include at least 30 wt% of a high (> 40,000 Mw or higher) PC copolymer having a THPE branch content of from 0.5-1.0 wt% and a reactive 4-HBN end-cap content of 2 mol% (CFR-PC1 and CFR-PC2). Even with 20 wt% of a lower Mw PC copolymer (CFR-PC or CFR-PC3), ductility is still 100% with similar flow (HDT) properties.

Surprisingly, good impact performance was observed in example composition E3 which includes 50 wt% PC copolymer components including a 55,000 g/mol Mw copolymer (30 wt% CFR-PC1) and a 28,000 g/mol Mw copolymer (20 wt% CFR-PC).

With the method outlined above, tough 4-HBN/THPE branched PC copolymer compositions are predictable and it is anticipated that fully colorable light diffused tough blends may be prepared.

The above description is intended to be illustrative, and not restrictive. For example, the above-described examples (or one or more aspects thereof) may be used in combination with each other. Other aspects can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed aspect. Thus, the following claims are hereby incorporated into the Detailed Description as examples or aspects, with each claim standing on its own as a separate aspect, and it is contemplated that such aspects can be combined with each other in various combinations or permutations. The scope of the disclosure should be determined with reference to the appended claims, along with the full scope of equivalents to which such claims are entitled.

## Claims

1. A thermoplastic composition comprising:

   a first branched polycarbonate copolymer end-capped with 4-hydroxybenzonitrile (4-HBN) having a molecular weight M1; and
   a second branched polycarbonate copolymer end-capped with 4-HBN having a molecular weight M2,
   wherein M1 is less than M2, and wherein molecular weight is evaluated according to polycarbonate standards.

2. The thermoplastic composition according to claim 1, wherein M1 is in a range of from 20,000 grams per mole (g/mol) to less than 35,000 g/mol, and wherein M2 is in a range of from 35,000 g/mol to 60,000 g/mol.

3. The thermoplastic composition according to claim 1 or 2, wherein each of the first branched polycarbonate copolymer and the second branched polycarbonate copolymer independently comprise from 1-8 mol% 4-HBN content.

4. The thermoplastic composition according to any of claims 1 to 3, wherein the composition further comprises (1) a third branched polycarbonate copolymer end-capped with 4-HBN, and (2) a polycarbonate homopolymer or an additional polycarbonate copolymer.

5. The thermoplastic composition according to claim 4, wherein the composition comprises at least 90 wt% of the first branched polycarbonate copolymer, the second branched polycarbonate copolymer, the third branched copolymer end-capped with 4-HBN, and the polycarbonate homopolymer or additional polycarbonate copolymer.

6. The thermoplastic composition according to any of claims 1 to 5, wherein one or more of the first branched polycarbonate copolymer, the second branched polycarbonate copolymer, and the at least one additional polycarbonate component comprise a 1,1,1-tris-hydroxy-phenylethane (THPE) branching agent.

7. The thermoplastic composition according to any of claims 1 to 6, wherein the composition comprises at least one additional additive comprising a flame retardant (FR) additive, a release agent, a heat stabilizer, a light stabilizer, a light diffuser, an anti-drip agent, a filler, or a combination thereof.

8. The thermoplastic composition according to any of claims 1 to 7, wherein the first branched polycarbonate copolymer or the second branched polycarbonate copolymer comprises:

an entanglement density in a range of from $2\times10^{-4}$ moles per cubic centimeter ($mol/cm^3$) to $10\times10^{-4}$ $mol/cm^3$;
a plateau modulus of from 1.0 to 4.0 megapascals (MPa); or
an entanglement molecular weight of from 1500 to 5000.

9. The thermoplastic composition according to any of claims 1 to 8, wherein a molded sample of the composition has a total light transmittance of at least 90% and a haze of 2% or less as tested in accordance with ASTM D1003 using a sample having a thickness of 3.2 mm.

10. A method for determining entanglement molecular weight (M*e*) of a polycarbonate copolymer, comprising:

preparing a sample of a branched polycarbonate copolymer comprising a 1,1,1-tris-hydroxy-phenylethane (THPE) branching agent and that is end-capped with 4-hydroxybenzonitrile (4-HBN);
determining phase angle ($\partial$) versus complex modulus (G*) of the sample using a small amplitude oscillatory rheology process at a specified temperature;
determining a minima in the $\partial$ of the sample;
determining a plateau modulus ($G_N$) of the sample from the determined minima of $\partial$; and
calculating the Me of the sample from the $G_N$.

11. The method according to claim 10, wherein the small amplitude oscillatory rheology process comprises:

melting the sample at 300 °C in a rheometer;
compressing the melted sample between parallel plates having a 1 millimeter (mm) gap;
cooling the parallel plates in the rheometer to the specified temperature, wherein the specified temperature is at least 15 °C above a glass transition temperature (Tg) of the branched polycarbonate copolymer; and
determining the phase angle ($\partial$) versus complex modulus (G*) of the sample at the specified temperature.

12. The method according to claims 10 or 11, further comprising predicting impact strength of the sample by comparing the Me of the sample to a reference table showing M*e* versus notched Izod impact strength for a plurality of branched polycarbonate copolymers comprising a 1,1,1-tris-hydroxy-phenylethane (THPE) branching agent and that are end-capped with 4-hydroxybenzonitrile (4-HBN).

13. The method according to any of claims 10 to 12, wherein the specified temperature is from 20-40 °C above the Tg of the branched polycarbonate copolymer.

14. The method according to any of claims 10 to 13, wherein the branched polycarbonate copolymer comprises from 0.5 to 1 mole percent THPE and has a 4-HBN end-cap level 2 mol% or less.

15. An article comprising the thermoplastic composition according to any of claims 1 to 9 wherein the article is a component of a consumer electronics or lighting appliance device.

Chemical Structure of CFR Polycarbonate Copolymer

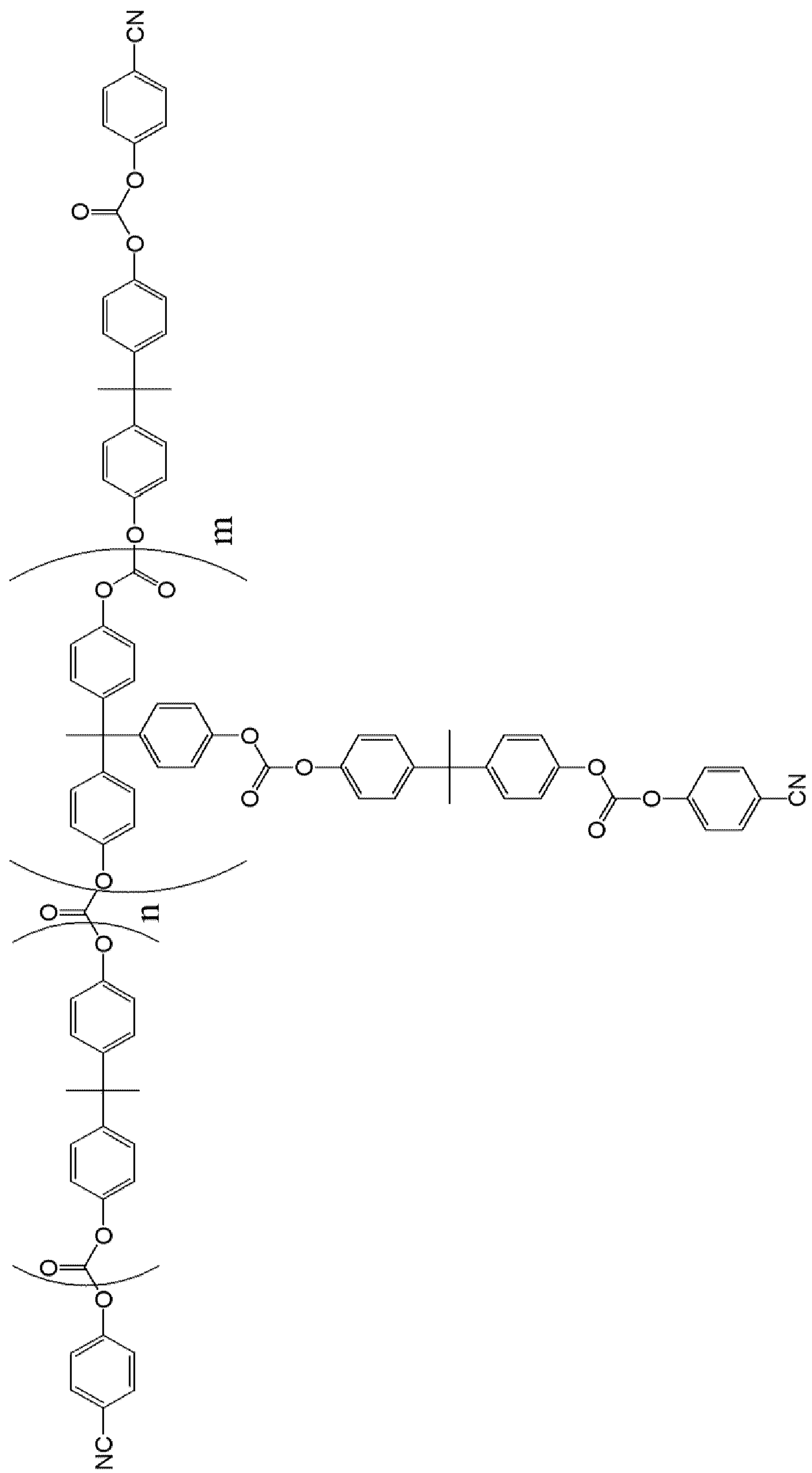

FIG. 1

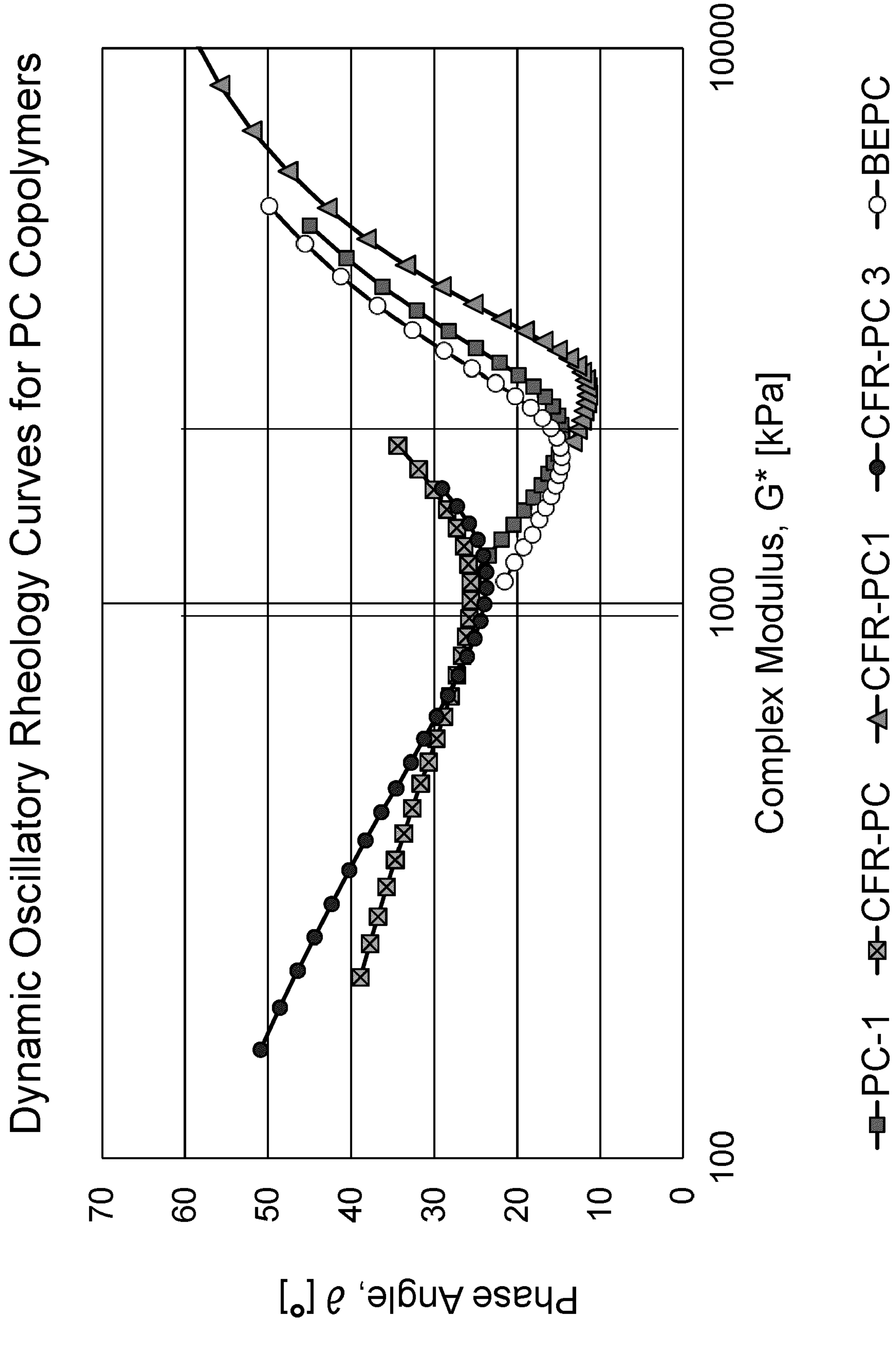
Dynamic Oscillatory Rheology Curves for PC Copolymers
Phase Angle, ∂ [°]
0
10
20
30
40
50
60
70
100
1000
10000
Complex Modulus, G* [kPa]
PC-1
CFR-PC
CFR-PC1
CFR-PC 3
BEPC

FIG. 2

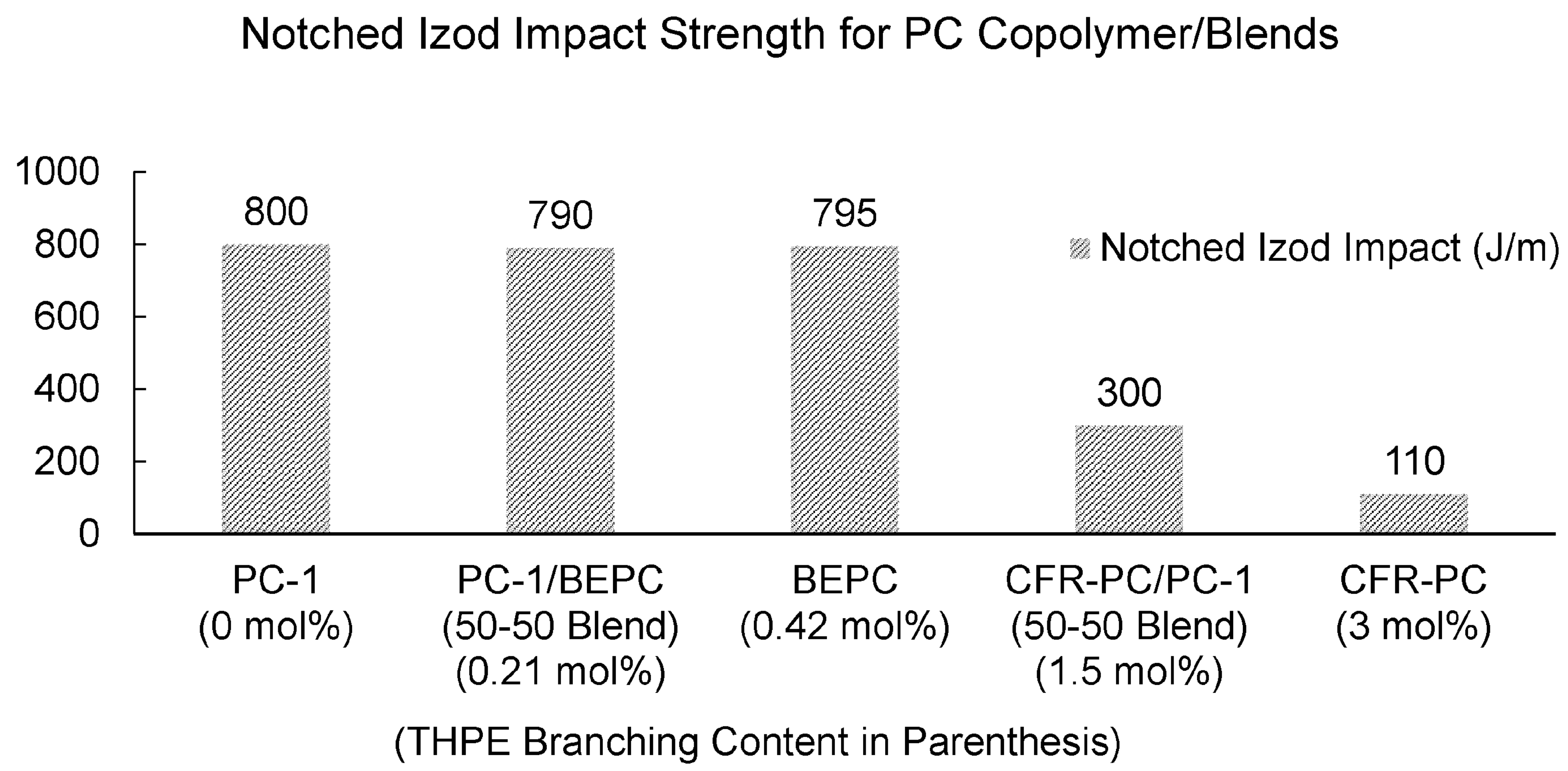
Notched Izod Impact Strength for PC Copolymer/Blends
1000
800
600
400
200
0
800
790
795
300
110
Notched Izod Impact (J/m)
PC-1 (0 mol%)
PC-1/BEPC (50-50 Blend) (0.21 mol%)
BEPC (0.42 mol%)
CFR-PC/PC-1 (50-50 Blend) (1.5 mol%)
CFR-PC (3 mol%)
(THPE Branching Content in Parenthesis)

FIG. 3

Europäisches Patentamt
European Patent Office
Office européen des brevets

# EUROPEAN SEARCH REPORT

Application Number
EP 23 16 3241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 736 132 A1 (SABIC GLOBAL TECHNOLOGIES BV [NL]) 11 November 2020 (2020-11-11)<br>* claims 1-14 *<br>* table 7 *<br>* example 10; table 8 *<br>----- | 1-9,15 | INV.<br>C08G64/18<br>C08G64/42 |
| A | US 2017/362432 A1 (MORIZUR JEAN-FRANCOIS [US] ET AL) 21 December 2017 (2017-12-21)<br>* claims 1-20 *<br>* table 1 *<br>* examples 5,7,8,12,13; tables 2,3 *<br>----- | 1-9,15 | |
| A | US 2020/308398 A1 (VAN DER MEE MARK ADRIANUS JOHANNES [NL] ET AL) 1 October 2020 (2020-10-01)<br>* claims 1-6 *<br>* paragraph [0087] *<br>* table 3 *<br>----- | 10-14 | |
| A | CN 102 159 643 A (SABIC INNOVATIVE PLASTICS IP) 17 August 2011 (2011-08-17)<br>* claims 1-4, 12 *<br>* paragraphs [0049] - [0050] *<br>----- | 10-14 | TECHNICAL FIELDS SEARCHED (IPC)<br>C08G<br>C09J |

The present search report has been drawn up for all claims

| Place of search | Date of completion of the search | Examiner |
|---|---|---|
| The Hague | 12 October 2023 | Mensah, Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

4

Europäisches Patentamt
European Patent Office
Office européen des brevets

Application Number
EP 23 16 3241

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [x] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 23 16 3241

The Search Division considers that the present European patent application does not comply with the requirements of unity of invention and relates to several inventions or groups of inventions, namely:

**1. claims: 1-9, 15**

**A thermoplastic composition comprising: a first branched polycarbonate copolymer end-capped with 4-hydroxybenzonitrile (4-HBN) having a molecular weight M1; and a second branched polycarbonate copolymer end-capped with 4-HBN having a molecular weight M2, wherein M1 is less than M2, and wherein molecular weight is evaluated according to polycarbonate standards, and article comprising the thermoplastic composition**

---

**2. claims: 10-14**

**A method for determining entanglement molecular weight (Me) of a polycarbonate copolymer, comprising: preparing a sample of a branched polycarbonate copolymer comprising a 1,1,1-tris-hydroxy-phenylethane (THPE) branching agent and that is end-capped with 4-hydroxybenzonitrile (4-HBN); determining phase angle (3) versus complex modulus (G*) of the sample using a small amplitude oscillatory rheology process at a specified temperature; determining a minima in the a of the sample; determining a plateau modulus (GN) of the sample from the determined minima of 8; and calculating the Me of the sample from the Grr.**

---

## ANNEX TO THE EUROPEAN SEARCH REPORT ON EUROPEAN PATENT APPLICATION NO. EP 23 16 3241

This annex lists the patent family members relating to the patent documents cited in the above-mentioned European search report. The members are as contained in the European Patent Office EDP file on
The European Patent Office is in no way liable for these particulars which are merely given for the purpose of information.

12-10-2023

| Patent document cited in search report | | Publication date | Patent family member(s) | | | Publication date |
|---|---|---|---|---|---|---|
| EP 3736132 | A1 | 11-11-2020 | CN | 114144300 | A | 04-03-2022 |
| | | | EP | 3736132 | A1 | 11-11-2020 |
| | | | KR | 20210152574 | A | 15-12-2021 |
| | | | US | 2022089900 | A1 | 24-03-2022 |
| | | | WO | 2020226774 | A1 | 12-11-2020 |
| US 2017362432 | A1 | 21-12-2017 | EP | 3268431 | A1 | 17-01-2018 |
| | | | US | 2017362432 | A1 | 21-12-2017 |
| | | | WO | 2016144309 | A1 | 15-09-2016 |
| US 2020308398 | A1 | 01-10-2020 | CN | 111793341 | A | 20-10-2020 |
| | | | EP | 3719066 | A1 | 07-10-2020 |
| | | | US | 2020308398 | A1 | 01-10-2020 |
| CN 102159643 | A | 17-08-2011 | CN | 102159643 | A | 17-08-2011 |
| | | | EP | 2342281 | A1 | 13-07-2011 |
| | | | US | 2010280159 | A1 | 04-11-2010 |
| | | | WO | 2010036900 | A1 | 01-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7786246 B


### Non-patent literature cited in the description


- *CHEMICAL ABSTRACTS,* 80-07-7
- *CHEMICAL ABSTRACTS,* 27955-94-8
- *CHEMICAL ABSTRACTS,* 767-00-0